# EUROPEAN PATENT SPECIFICATION

(11) **EP 2 307 056 B1**
(45) Date of publication and mention of the grant of the patent: **15.12.2021**
(21) Application number: 09810864.0
(22) Date of filing: 12.01.2009
(51) Int. Cl.: A61K 9/00, A61K 47/40, A61K 47/02, A61K 31/167, A61K 33/04, A61K 31/724, A61P 29/00, A61K 9/19

(54) **STABILIZED AQUEOUS FORMULATION CONTAINING PARACETAMOL**
STABILISIERTE WÄSSRIGE FORMULIERUNG MIT PARACETAMOL
COMPOSITIONS PHARMACEUTIQUES AQUEUSES STABLES CONTENANT DU PARACETAMOL

(30) Priority: 17.01.2008 IN CH01472008
(43) Date of publication of application: 13.04.2011
(73) Proprietor: Pharmis Biofarmacêutica, LDA, 2750-341 Cascais (PT)
(72) Inventor: KANDHAGATLA, Rajnarayana, Hyderabad 500 034 (IN); KALIDINDI, Srihari Raju, Hyderabad 500 034 (IN)
(74) Representative: Hamm&Wittkopp Patentanwälte PartmbB
(86) International application number: PCT/IN2009/000038
(87) International publication number: WO 2009/098716

(56) References cited:
- EP-A1- 1 752 139
- WO-A1-90/13290
- FR-A1- 2 751 875
- FR-A1- 2 751 875
- US-A- 4 727 064
- US-A1- 2005 215 520
- None

## Description

### Field of the invention

The present invention relates to stable pharmaceutical aqueous compositions, having analgesic activity comprising a therapeutically effective dose of paracetamol, the processes for preparing the same and methods of use and treatment of such compositions.

### Background of the invention

Paracetamol (Formula I) is chemically N- (4-Hydroxyphenyl)acetamide. It is an analgesic and antipyretic agent. The mechanism of analgesic action of paracetamol has not been fully determined. Paracetamol may act predominantly by inhibiting prostaglandin synthesis in the central nervous system and to a lesser extent through a peripheral action by blocking pain impulse generation. The peripheral action may also be due to inhibition of prostaglandin synthesis or inhibition of the synthesis or actions of other substances that sensitise pain receptors. In recommended therapeutic dosage, it is usually well tolerated. However, acute overdose may cause fatal hepatic damage. The potentially toxic metabolite is said to be N-acetyl-p-benzoquinoneimine.

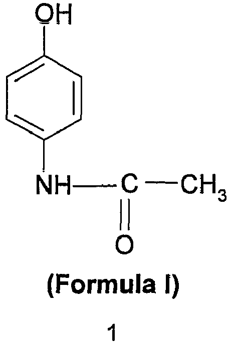

Formulating liquid compositions of paracetamol has critical considerations.The paracetamol in aqueous solution is liable to undergo hydrolysis to form p-aminophenol, which itself degrades to quinoneimine. Also, the stablility of aqueous formulations is dependent on variables such as removal of dissolved oxygen from carrier, optional further presence of antioxidant in the formulation and adjustment to suitable pH. Hence a composition comprising an aqueous formulation of paracetamol wherein the stability of the formulation is achieved is highly desirable.

Perfalgan^{®} is a commercially available formulation which comprises a liquid formulation of paracetamol. The formulation contains buffering agents. It is available from Bristol Myers Squibb.

US Pat No. 6992218 reports a method for preparing aqueous formulations with easily oxidizable active principles, notably phenols. The method comprises deoxygenation of the solution by bubbling it with at least one inert gas until the oxygent content is below 2 ppm.

The preparation of parenteral formulations of paracetamol has been known in the art. However, the parenteral formulations of paracetamol utilize a buffer system to maintain the pH to the required value (FR 2751875 A1, US Pat No. 6028222).

EP 1752139 describes liquid formulations of paracetamol for infusion, without using a buffer system. However, the formulations comprise antioxidants selected from a group of ascorbic acid, N-acetyl-L-cysteine and thiol group-containing stabilizer compounds.

The use of organic solvents for the preparation of liquid formulations of paracetamol has also been reported (WO 05053747, WO 0007588). However, the prior art stabilized liquid formulation of paracetamol have the drawback of causing a potential irritant and/or allergic effect in certain patients, because of the toxicity of either the antioxidant or the organic solvent present in the formulation.

Hence, the development of an aqueous parenteral formulation of paracetamol which does not contain a buffer system or an organic solvent and bypasses all the disadvantages mentioned above would be a significant improvement in the field of medical practice. Thus the present invention fills a highly desirable gap in medical art comprising stable aqueous formulations of paracetamol.

### Summary of the invention

The objective of the present invention is to provide stable parenteral compositions of paracetamol, wherein the paracetamol formulation does not contain a buffer system.

Further it is an objective of the present invention to provide aqueous compositions of paracetamol, having analgesic activity, which are formulated without any organic solvent and hence to bypass any allergic, irritant or similar potential side effect caused by such solvents.

### Detailed description of the invention

The present invention relates to aqueous, parenteral paracetamol compositions which comprise a therapeutically effective dose of paracetamol. The paracetamol compositions as per the present invention are formulated without the addition of any organic solvent and do not contain a buffer system.

In an embodiment, stable pharmaceutical aqueous compositions as disclosed in the invention are formulated as parenteral formulations, in particular infusible formulations, in a solvent.

In another embodiment of the present invention, the preferable solvent is water.

The stable pharmaceutical aqueous compositions of the present invention include a solubilising aid, namely hydroxy propyl beta cyclodextrin.

By 'solubilising aid' is meant any compound that may assist in solubilising the active by accommodating the active in a cavity formed in the solubising aid to form inclusion complexes.

Hydroxy propyl beta cyclodextrin, herein referred to as "HPβCD", was found to be useful in the context of the present invention.

The w/w ratios of HPβCD to the active can range from about 0.5:1 to about 2:1, particularly from about 0.75:1 to about 1.5:1.

The present invention provides stable pharmaceutical aqueous compositions, having analgesic activity, comprising a therapeutically effective dose of paracetamol with the solubilising aid and sodium metabisulfite as antioxidant.

Sodium metabisulfite may be used in amount from about 200 mg/l to about 1g/l.

In addition to the use of the antioxidant, the antioxidant effect is achieved by displacing oxygen from the composition of the active(s). This may be achieved by purging the composition with a water insoluble inert gas. Inert gases which are known to a person skilled in the art may

be employed. These include but are not limited to nitrogen, carbon dioxide and the like.

In an embodiment, the pharmaceutical composition was purged with nitrogen to displace the oxygen from the composition.

The oxygen content in the composition of the invention is not more than 2 ppm.

Common isotonic agents may be used in the pharmaceutical compositions of the present invention to impart same osmotic pressure as the body fluid to the formulations. Isotonicity of the preparation may be achieved by adding an appropriate quantity of isotonic agent. Suitable isotonic agents which may be used in the context of the above invention may include but are not limited to sodium chloride, calcium chloride, potassium chloride, glucose, levulose and the like.

In an embodiment, sodium chloride was found to be useful in the context of the present invention.

The aqueous parenteral formulation of paracetamol, in the context of the present invention contains no buffer system. The pH of the formulation, according to the invention may be adjusted using chemicals such as sodium hydroxide, hydrochloric acid and the like to a value of about 4 to 8.

In an embodiment, the pH of the formulation according to the invention was adjusted using NaOH.

In another embodiment, the pH of the formulation according to the present invention may be between about 4 to about 8, for example about 4.5 to about 7.5, typically about 4.5 to about 6.5 and more typically about 5.5 to about 6.5.

Parenteral formulations according to the invention may comprise other excipients commonly employed in parenteral formulations for intravenous administration in order to provide the required stability and/or therapeutic efficacy. This may include any other excipients commonly used in the preparation of parenteral formulations for intravenous administration.

The pharmaceutical compositions according to the said invention may comprise other agents which may enhance and/ or potentiate the effect of paracetamol.

The aqueous paracetamol formulation according to the present invention can be used in the field of medicine as a parenteral analgesic.

The aqueous composition of the present invention may be prepared by processes known to one skilled in the art, e.g. by the following process:
a) Dissolving either paracetamol or the solubilising aid or both in the solvent or mixture of solvents optionally with other pharmaceutically acceptable excipients optionally under inert atmosphere.
b) Optionally adjusting the pH and/or the osmolality of the solution to suitable values.
c) Subjecting the solution to sterilization optionally using filteration sterilization.
d) Optionally deoxygenating the resultant solution using inert gas and/ or optional terminal sterilization.

### Examples

The following examples are included to illustrate certain aspects of the invention in greater detail but are not intended to limit the scope of the invention in any way.

### Example 1

### Composition of Paracetamol solution

| S. No | Ingredients | Amount |
|---|---|---|
| 1 | Sodium metabisulfite | 10.00 mg |
| 2 | Hydroxypropyl betacyclodextrin (HPβCD) | 150.00 mg |
| 3 | Paracetamol | 100.00 mg |
| 4 | Sodium chloride | 63.00 mg |
| 5 | Sodium hydroxide | q.s to pH 5.5 |
| 6 | Water for injection | q.s to 10.00 ml |

### Manufacturing procedure:

Required amounts of sodium metabisulfite, HPβCD, paracetamol and sodium chloride were dissolved in water for injection, with stirring under inert atmosphere, until each of the ingredients dissolved completely. After a clear solution was obtained, the volume was made up with water for injection and the pH of the resultant solution was adjusted with sodium hydroxide. After filtration through a sterile 0.22 µm pore size filter, the solution was purged with inert gas such as nitrogen and subsequently filled in Type II glass vials. The vials were sealed with rubber closure and aluminium caps and sterilized by autoclaving at 121°C/15 lbs for 15 minutes. 1lbs = 0.4535kg.

### Example 2

### Composition of Paracetamol solution

| S. No | Ingredients | Amount |
|---|---|---|
| 1 | Sodium metabisulfite | 10.00 mg |
| 2 | Hydroxypropyl betacyclodextrin (HPβCD) | 150.00 mg |

| | | |
|---|---|---|
| 3 | Paracetamol | 100.00 mg |
| 4 | Sodium chloride | 63.00 mg |
| 5 | Sodium hydroxide | q.s to pH 5.5 |
| 6 | Water for injection | q.s to 10.00 ml |

### Manufacturing procedure:

Required amounts of sodium metabisulfite, HPβCD, paracetamol and sodium chloride were dissolved in water for injection, with stirring under inert atmosphere, until each of the ingredients dissolved completely. After a clear solution was obtained, the volume was made up with water for injection and the pH of the resultant solution was adjusted with sodium hydroxide. After filtration through a sterile 0.22 µm pore size filter, the solution was purged with inert gas such as nitrogen and subsequently filled in Type II glass vials. The vials were sealed with rubber closure and aluminium caps and sterilized by autoclaving at 105°C/8 lbs for 30 minutes. 1lbs = 0.4535kg.

The vials each from Example 1 and Example 2 were stored at 25° ± 2°C and 60% ± 5% RH as well as 40° ± 2°C and 75% ± 5% RH. Stability data for the vials stored at 25° ± 2°C as well as 60% ± 5% RH and 40°C and 75% RH indicated that the vials were stable and clear of coloured particles for at least six months. The vials each from Example 1 and Example 2 were subjected to the evaluation of the following parameters:
1. Paracetamol assay: (%) By High Performance Liquid Chromatography (HPLC)
2. p- amino phenol titre: (%) By HPLC
3. Total Impurities: (%) By HPLC
4. Dissolved Oxygen: (ppm) By Dissolved oxygen meter

### Vials from Example 1 stored at 25° ± 2°C and 60% ± 5% RH

| | Sampling time | | |
|---|---|---|---|
| | Initial (Time 0) | 3 months | 6 months |
| Paracetamol assay | 102.4 | 100.8 | 101.0 |
| p- amino phenol | 0.008 | 0.007 | 0.01 |
| Total Impurities | 0.022 | 0.025 | 0.036 |
| Dissolved oxygen | 0.38 | 0.40 | 0.65 |

### Vials from Example 1 stored at 40° ± 2°C and 75% ± 5% RH

| | Sampling time | | |
|---|---|---|---|
| | Initial (Time 0) | 3 months | 6 months |
| Paracetamol assay | 102.4 | 100.4 | 100.3 |
| p- amino phenol | 0.018 | 0.017 | 0.02 |
| Total Impurities | 0.027 | 0.061 | 0.063 |
| Dissolved oxygen | 0.38 | 0.85 | 1.01 |

### Vials from Example 2 stored at 25° ± 2°C and 60% ± 5% RH

| | Sampling time | | |
|---|---|---|---|
| | Initial (Time 0) | 3 months | 6 months |
| Paracetamol assay | 102.6 | 102.2 | 101.8 |
| p- amino phenol | BDL* | 0.009 | 0.015 |
| Total Impurities | 0.024 | 0.028 | 0.047 |
| Dissolved oxygen | 0.38 | 0.31 | 0.89 |

| | | | |
|---|---|---|---|
| * BDL- Below detectable limit | | | |

### Vials from Example 2 stored at 40° ± 2°C and 75% ± 5% RH

| | Sampling time | | |
|---|---|---|---|
| | Initial (Time 0) | 3 months | 6 months |
| Paracetamol assay | 102.6 | 101.4 | 101.0 |
| p- amino phenol | BDL | 0.017 | 0.019 |
| Total Impurities | 0.014 | 0.050 | 0.058 |
| Dissolved oxygen | 0.38 | 0.52 | 0.89 |

## Claims

1. An aqueous, stable parenteral paracetamol formulation comprising:
(i) hydroxy propyl beta cyclodextrin;
(ii) sodium metabisulfite as antioxidant;
**characterized in that**:
(a) it has no buffering agent;
(b) it has no organic solvent;
(c) it exhibits an oxygen content of not more than 2 ppm.

2. The formulation of claim 1, containing an isotonicity agent.

3. The formulation of claim 2, wherein the isotonicity agent is sodium chloride.

4. The formulation of any of the preceding claims, wherein the pH of the composition is 4-8.

5. The formulation of claim 4, wherein the pH of the composition is 5.5-6.5.

6. A method of preparing the formulation according to any of the preceding claims comprising dissolving paracetamol together with hydroxy propyl beta cyclodextrin and the antioxidant in water, optionally along with an isotonicity agent.

7. The formulation according to any of claims 1 to 5 for use as a parenteral analgesic.

## Patentansprüche

1. Wässrige, stabile parenterale Paracetamol-Formulierung umfassend:
(i) Hydroxypropyl-beta-cyclodextrin;
(ii)Natriumdisulfit als Antioxidans;
**dadurch gekennzeichnet, dass**:
(a)sie keine Puffersubstanz enthält;
(b)sie kein organisches Lösungsmittel enthält;
(c)sie einen Sauerstoffgehalt von nicht mehr als 2 ppm aufweist.

2. Formulierung gemäß Anspruch 1, enthaltend ein Tonizitätsmittel.

3. Formulierung gemäß Anspruch 2, wobei das Tonizitätsmittel Natriumchlorid ist.

4. Formulierung gemäß einem der vorstehenden Ansprüche, wobei der pH-Wert der Zusammensetzung 4-8 ist.

5. Formulierung gemäß Anspruch 4, wobei der pH-Wert der Zusammensetzung 5,5-6,5 ist.

6. Verfahren zur Herstellung einer Formulierung gemäß einem der vorstehenden Ansprüche, umfassend Lösen von Paracetamol zusammen mit Hydroxypropyl-beta-cyclodextrin und dem Antioxidans in Wasser, gegebenenfalls zusammen mit einem Tonizitätsmittel.

7. Formulierung gemäß einem der Ansprüche 1-5 zur Verwendung als parenterales Analgetikum.

## Revendications

1. Formulation de paracétamol aqueuse, stable parentérale comprenant :
(i) hydroxypropyl-bêta-cyclodextrine ;
(ii) métabisulfite de sodium en tant qu'antioxydant ;
**caractérisée en ce que** :
(a) elle n'a aucun agent tampon ;
(b) elle n'a aucun solvant organique ;
(c) elle présente une teneur en oxygène ne dépassant pas 2 ppm.

2. Formulation selon la revendication 1, contenant un agent d'isotonicité

3. Formulation selon la revendication 2, dans laquelle l'agent d'isotonicité est du chlorure de sodium.

4. Formulation selon l'une quelconque des revendications précédentes, dans laquelle le pH de la composition est de 4 à 8.

5. Formulation selon la revendication 4, dans laquelle le pH de la composition est de 5,5 à 6,5.

6. Procédé de préparation de la formulation selon l'une quelconque des revendications précédentes, comprenant la dissolution de paracétamol ensemble avec de l'hydroxypropyl-bêta-cyclodextrine et l'antioxydant dans de l'eau, facultativement conjointement avec un agent d'isotonicité.

7. Formulation selon l'une quelconque des revendications 1 à 5 à utiliser comme analgésique parentéral.
